(19) **Europäisches Patentamt / European Patent Office / Office européen des brevets**

(11) **EP 3 574 306 B1**

(12) **EUROPEAN PATENT SPECIFICATION**

(45) Date of publication and mention
of the grant of the patent:
**03.03.2021 Bulletin 2021/09**

(21) Application number: **18704085.2**

(22) Date of filing: **25.01.2018**

(51) Int Cl.:
*G01N 21/25* $^{(2006.01)}$          *G01N 33/50* $^{(2006.01)}$
*G01N 33/569* $^{(2006.01)}$

(86) International application number:
**PCT/IB2018/050440**

(87) International publication number:
**WO 2018/138659 (02.08.2018 Gazette 2018/31)**

(54) **A METHOD AND SYSTEM FOR ANALYSING A BIOLOGICAL SAMPLE OF LABEL-FREE CELLS FOR PRESENCE OF AN INFECTIVE AGENT**

VERFAHREN UND SYSTEM ZUR ANALYSE EINER BIOLOGISCHEN PROBE VON MARKIERUNGSFREIEN ZELLEN AUF ANWESENHEIT EINER INFEKTIÖSEN SUBSTANZ

PROCÉDÉ ET SYSTÈME D'ANALYSE D'UN ÉCHANTILLON BIOLOGIQUE DE CELLULES SANS MARQUEURS POUR DÉTECTER LA PRÉSENCE D'UN AGENT INFECTIEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2017 ZA 201700578**

(43) Date of publication of application:
**04.12.2019 Bulletin 2019/49**

(73) Proprietor: **CSIR**
**0002 Pretoria (ZA)**

(72) Inventors:
• **OMBINDA LEMBOUMBA, Saturnin**
**0001 Pretoria (ZA)**
• **MALABI, Rudzani**
**0001 Pretoria (ZA)**
• **MTHUNZI-KUFA, Patience**
**0001 Pretoria (ZA)**
• **LUGONGOLO, Masixole, Yvonne**
**0001 Pretoria (ZA)**

• **THOBAKGALE, Lebogang**
**0001 Pretoria (ZA)**

(74) Representative: **D Young & Co LLP**
**120 Holborn**
**London EC1N 2DY (GB)**

(56) References cited:
**WO-A1-03/093496     DE-A1-102014 003 386**

• HAMDEN ET AL: "Spectroscopic analysis of Kaposi's sarcoma-associated herpesvirus infected cells by Raman tweezers", JOURNAL OF VIROLOGICAL MET, ELSEVIER BV, NL, vol. 129, no. 2, 29 June 2005 (2005-06-29), pages 145-151, XP005087821, ISSN: 0166-0934, DOI: 10.1016/J.JVIROMET.2005.05.018
• LAMBERT PHELPS J ET AL: "Raman spectroscopy: the gateway into tomorrow's virology", VIROLOGY JOURNAL, BIOMED CENTRAL, LONDON, GB, vol. 3, no. 1, 28 June 2006 (2006-06-28), page 51, XP021019356, ISSN: 1743-422X, DOI: 10.1186/1743-422X-3-51

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## FIELD OF INVENTION

This invention relates to an *ex vivo* method for analysing a biological sample of label-free cells for the presence of an infective agent (an HIV virus) in or attached to the cell. Also described is a system for carrying out the method.

## BACKGROUND OF INVENTION

Diagnostics tools such as enzyme-linked immunosorbent assay (ELISA) and nucleic acid-based tests are associated with inaccurate results, require labelling and substrates respectively, and are unable to produce results in real-time. They are consequently not well suited for point-of-care (POC) diagnostics in resource limited environments and there is a need to develop a new diagnostics tool that is rapid, accurate and overcomes at least some of these challenges.

The need to implement a label-free technique for the detection of biological agents has prompted the identification of different methods to investigate the underlying physical and chemical characteristics of biological samples whilst reducing the complexity and preparation time. Spectroscopy, and in particular transmission spectroscopy, has been identified as a potential candidate for label-free investigation of biological material. It is a real-time and non-invasive optical detection technique that has wide application in the pharmaceutical industry as well as in the biological and medical fields. Transmission spectra patterns have been used, for instance, to differentiate red from white human blood cells and cancerous from non-cancerous cell species. A drawback of conventional transmission spectroscopy is that it is carried out on an ensemble averaging measurement of cells and the ensemble measurement yields information on the average properties. As a result, the information and properties of a single cell are overshadowed by averaging measurements. Further, with transmission spectroscopy, sample thickness and sample inhomogeneity can cause severe loss of the transmitted signal. Transmission measurements are often done on immobilised cells using substrate or aqueous cell suspensions where samples are not uniformly distributed and are therefore inhomogeneous, which can generate spectral artefacts. These artefacts generally alter the peak intensity and lead to a false interpretation of results. The present invention is therefore directed at stably isolating and studying a single cell in real-time.

Optical trapping is an invaluable tool for the micromanipulation of viruses, bacteria and cells suspended in liquid media. This technique essentially uses the momentum of a light particle to hold, orient and transport a single particle or cell at will via the force of the radiation pressure from a tightly focused laser beam emitted through a high numerical aperture objective lens. This cell immobilisation allows for accurate investigation of individual cells. The use of near infrared lasers for optical trapping has enabled non-invasive manipulation of non-adherent cells in a label-free fashion.

HAMDEN ET AL: "Spectroscopic analysis of Kaposi's sarcoma-associated herpesvirus infected cells by Raman tweezers", JOUIRNAL OF VIROLOGICAL MET, ELSEVIER BV, NL, vol. 129, no. 2, 29 June 2005, pages 145 -151 discloses the use of Raman tweezers to identify KSHV-infected cells.

LAMBERT PHELPS J ET AL: "Raman spectroscopy: the gateway into tomorrow's virology", VIROLOGY JOURNAL, BIOMED CENTRAL, LONDON, GB, vol 3, no. 1, 28 June 2006, page 51 is a review paper that broadly discloses Raman spectroscopy and its potential for use in virology.

DE 10 2014 003386 A1 discloses a method and device using Raman spectroscopy for quality control of a blood-based product.

WO 03/093496 A1 teaches an optophoretic method to determine a biological property of a cell.

None of these documents discloses a method for analysing a biological sample for viral infected cells using an optical trap laser beam in combination with absorption or transmission spectroscopy.

## SUMMARY OF INVENTION

The invention provides an *ex vivo* method of analysing a biological sample comprising a plurality of label-free cells to determine whether or not the sample includes cells that are infected by an HIV virus, the method including:

optically trapping a label-free cell in the biological sample with an optical beam; and
spectroscopically investigating the trapped label-free cell to determine in real time if the trapped label-free cell is infected by an HIV virus, wherein spectroscopically investigating the trapped label-free cell includes determining a transmission value or an absorption value of the label-free cell, and wherein the transmission value or absorption value is indicative of whether or not the label-free cell is infected by the HIV virus.

By "label-free" is meant a cell that does not include a marker, such as an enzyme, radioactive isotope, DNA probe, fluorogenic reporter, electrochemiluminescent tag, or other foreign element, that is conventionally used to detect the presence of an infective agent in a cell.

Optical trapping, also known as "optical tweezing", first reported by Arthur Ashkin in 1970, is by now a well-known non-contact technique to hold a microscopic particle stable. Optical trapping typically employs a highly focused laser beam (often referred to as an optical tweezer) to provide an attractive or repulsive force, typically of the order of piconewtons, physically to hold and/or move microscopic dielectric objects. The technology has been well described in literature, including its use to trap individual viruses and bacteria and to sort cells and colloids.

Spectroscopy is also a well-known analytical technique, relying on the interaction between matter and radiative energy, e.g. electromagnetic radiation, to characterise the matter.

To the best of the inventors' knowledge, optical trapping, particularly in combination with spectroscopy, has not yet been used to determine whether or not a biological sample includes label-free cells that are infected by an infective agent, such as a virus.

The optical beam may be a laser beam, e.g. a Nd:YAG (Neodymium-doped Yttrium Aluminium Garnet) laser beam with a wavelength of 1064 nm.

The HIV virus is the human immunodeficiency virus (HIV) that causes HIV infection and over time, acquired immunodeficiency syndrome (AIDS). In at least one embodiment of the invention, the HIV virus may be HIV-1.

The label-free cells may be suspended in a fluid, e.g. a liquid or solution.

Optically trapping a label-free cell in the biological sample may include trapping, and if desired, displacing, the label-free cell in a two-dimensional plane, or in a three-dimensional space. Typically, optically trapping a label-free cell in the biological sample includes trapping, and if desired, displacing, the label-free cell in a three-dimensional space.

In this specification, "in real-time" is intended to indicate that a result for a trapped label-free cell is available within a few minutes, say within less than 4 minutes or within less than 3 minutes or within less than 2 minutes or within less than 1 minute of starting the spectroscopic investigation of the trapped cell.

Spectroscopically investigating the trapped label-free cell may additionally indicate a degree of viral infection in the biological sample, e.g., a viral load.

The method may be implemented iteratively or repetitively, thereby to investigate a plurality of trapped label-free cells. Investigating the plurality of trapped label-free cells may increase an accuracy of the investigation or provide additional information or diagnosis.

Optically trapping the label-free cell and spectroscopically investigating the trapped label-free cell may each be done by a means of a laser beam. Optically trapping the label-free cell and spectroscopically investigating the trapped label-free cell may be done by a means of the same laser beam or different laser beams. Optically trapping the label-free cell and spectroscopically investigating the trapped label-free cell may be done simultaneously or sequentially.

Thus, spectroscopically investigating the trapped label-free cell to determine in real-time if the trapped label-free cell is infected by an HIV virus employs absorption or transmission spectroscopy to detect the presence or absence of \. HIV in a cell. In one embodiment of the invention, near infrared (typically in the region of 750 - 1400 nm light) transmission spectroscopy is employed.

The method may include generating a spectrogram representative of at least one spectroscopic characteristic of the trapped label-free cell. The spectrogram may show the average signal spectra from a plurality of measurements taken on a single trapped label-free cell. The spectrogram may be analysed using conventional spectroscopy techniques.

The method may include comparing the spectrogram against a condition threshold. For example, if a spectroscopic characteristic of the spectrogram (e.g., optical transmission) is one side of (e.g., below) the condition threshold, this may indicate presence HIV; if the spectroscopic characteristic (e.g., optical transmission) is the other side of (e.g., above) the condition threshold, this may indicate absence of HIV.

The condition threshold may be a pre-defined threshold. The condition threshold may be pre-defined based on prior analysis (e.g., statistical analysis) of prior biological samples wherein the presence or absence of HIV is known beforehand.

Also described herein is a system for analysing a biological sample comprising a plurality of label-free cells to determine if the sample includes cells infected by an infective agent, the system comprising a laser apparatus and an optical detector, wherein:

the laser apparatus is configured to emit an optical trap laser beam configured optically to trap and isolate a single label-free cell from the biological sample;
the laser apparatus is configured to emit a diagnostic laser beam towards the trapped label-free cell; and
the optical detector is configured to detect the diagnostic laser beam after interaction with the trapped label-free cell wherein at least one spectroscopic characteristic of the detected laser beam is available in real-time and indicative of whether or not the trapped label-free cell is infected by an infective agent.

The laser apparatus may be configured to emit the diagnostic laser beam at a wavelength selected such that it is partially transmitted through the trapped label-free cell.

The laser apparatus may include a single laser beam emitter, with the single laser beam emitter configured to emit both the optical trap laser beam and the diagnostic laser beam. The optical trap laser beam and the diagnostic laser beam may be emitted simultaneously. The optical trap laser beam and the diagnostic laser beam may be the same laser beam. The optical trap laser beam and the diagnostic laser beam may be emitted sequentially.

The laser apparatus may include a plurality of laser beam emitters, with one laser beam emitter configured to emit the optical trap laser beam and another laser beam emitter configured to emit the diagnostic laser beam. The optical trap laser beam and the diagnostic laser beam may be emitted simultaneously. The optical trap laser beam and the diagnostic laser beam may be emitted sequentially.

The laser apparatus may include at least one laser diode to emit the optical trap laser beam and/or the diagnostic laser beam. The laser diode may be an infrared laser diode. The laser diode may be configured to emit a continuous wave. The laser diode may have an emission wavelength of 1064 nm $\pm$ 10%. The laser diode may have an output power of 0.1-10 W, more specifically 0.5-3.0 W, e.g., 1.5 W.

The optical trap laser beam is preferably operated or configured such than no phototoxic and/or thermal damage is caused to the trapped label-free cell being investigated.

The optical trap laser beam may be a single beam gradient force trap.

The optical trap laser beam may have a cross-sectional area or a diameter that is less or smaller than the corresponding measurement of the label-free cell.

## BRIEF DESCRIPTION OF DRAWINGS

The invention will now be further described, by way of an Example, with reference to the accompanying diagrammatic drawings.

In the drawings:

FIGURE 1 shows, for the Example, an optical trapping spectroscopy experimental system to measure transmittance and absorption spectra of TZM-bl infected and uninfected single cells;

FIGURE 2 shows, for the Example, a procedure for recording the background, reference and transmitted signal;

FIGURE 3 shows, for the Example, transmission spectra recorded between 1060 nm and 1068 nm of cell growth media, cell growth media with HIV-1 virus and laser light at 1064 nm;

FIGURE 4 shows, for the Example, a sequence of images for optically trapped silica beads (A) before and (B),(C) after stage movement successively, trapping power 60 mW, arrows indicating movement of surrounding untrapped beads;

FIGURE 5 shows, for the Example, TZM-bl cells in suspension in a sample chamber with the trapped cell in the focus plane and untrapped cells appearing out of focus and blurry in the second plane;

FIGURE 6 shows, for the Example, optically trapped uninfected TZM-bl single cells (A) before and (B) after stage movement respectively, and HIV-1 infected (C) before and (D) after stage movement successively, with trapping wavelength of 1064 nm, power 60 mW, arrows indicating movement of surrounding untrapped cells;

FIGURE 7 shows, for the Example, transmission spectra at 9425 $cm^{-1}$ and 9375 $cm^{-1}$ of cell growth media and cell growth media with HIV-1 virus;

FIGURE 8 shows, for the Example, transmission spectra average data of individually trapped HIV-1 infected and uninfected cells suspended in cell growth media with full range spectra; and

FIGURE 9 shows, for the Example, transmittance spectra peak values of ten infected cells compared to ten uninfected cells.

## EXAMPLE

The following description of the invention is provided as an enabling teaching of the invention. Thus, the following description is provided as illustrative of the principles of the present invention and not a limitation thereof.

The present invention comprises a novel combination of an optical trapping system with laser transmission spectroscopy, using a single beam gradient trap to determine the spectral transmittance intensity changes that are associated with single, trapped, label-free HIV-1 infected cells versus single, trapped, label-free uninfected cells, and in so doing, identifying in real-time whether or not a trapped cell is infected with HIV-1.

### Materials and methods

### Cell culture

TZM-bl cells were cultured using a T-75 vented top culture flask (The Scientific Group, 430641U) in Dulbecco's Modified Eagle's Medium (DMEM) (Sigma-Aldrich, D5796,) supplemented with 10% Foetal Bovine Serum (FBS) (Biochrom, S0615) and 1% Penicillin-Streptomycin (Sigma-Aldrich, P4333) antibiotics. The cultured cells were incubated at 37°C, 5% $CO_2$ and 85% humidity (optimum conditions) incubator until 70-80% confluency was reached and subsequently trypsinised with trypsin ethylenediaminetetraacetic acid (EDTA) (Sigma-Aldrich, T4049) to the next passage.

### Sample preparation

Cells were seeded at a concentration of $2x10^4$ in 35 mm diameter glass bottom petri dishes with a 23 mm diameter type 1 glass coverslip for 48 hours in optimum conditions. Cells were cultured in two groups namely HIV infected and uninfected cells. After the incubation time the plates were washed with 1ml 1X Hanks Balanced Salt solution (HBSS) (Gibco). An equal volume of 500 $\mu$l of trypsin was added to the plates and incubated for 3 minutes, and 1 ml of 10 % growth media was further added to stop trypsinisation. The solution for each plate was transferred to a 2 ml Eppendorf tube where it was spun down at 2200 rpm via a microcentrifuge (Corning LSE high speed microcentrifuge 6767HS) for 5 minutes.

After discarding the supernatant, the cell pellet was resuspended in 500 $\mu$l of fresh growth media and a

volume of 50 $\mu$l of each group transferred to a new tube and topped up with 500 $\mu$l making a 1:10 dilution of resuspended cells to growth media mix. A total volume of 500 $\mu$l of each group was separately transferred into 35 mm glass bottom petri dishes covered with a glass coverslip type 1 prior to observation on the optical setup. For the purpose of transmittance experiments, intensity background subtractions and normalisation were first conducted by subtracting the background light from the laser intensity signal and have a laser intensity reference, thereafter the two groups of cells i.e. HIV-1 infected and uninfected cells were analysed.

## Optical trapping spectroscopy setup

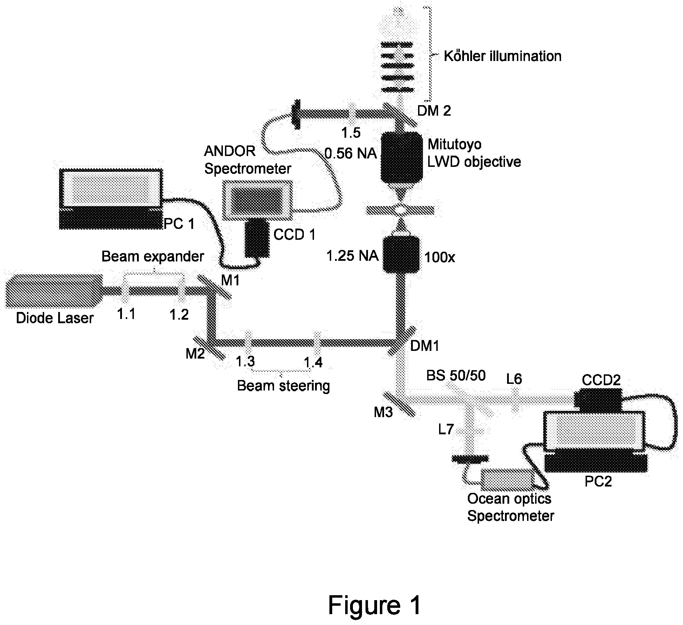

Figure 1 illustrates the optical trapping combined with a transmission spectroscopy system. In the system, L1-L7 are lenses, M1-M2 are infrared mirrors, M3 is a silver mirror, DM is a dichroic mirror, and BS is a beam splitter. The system utilises a tuneable single continuous wave (CW) diode laser beam with an output wavelength of 1064 nm producing a maximum output power of 1.5 W with a $M^2$ <1.2 and power stability of 0.108% (MIL-S-1064-A, Changcun New Industries, China). A 1 mm diameter Gaussian laser beam of 1.8 mrad divergence passes through a nine time beam expander (effectively embodying a telescope comprising two lenses having respective focal distances of 50 mm and 450 mm) in order slightly to overfill the back aperture of a 100x 1.25 NA oil immersion microscope objective.

A beam steering lens relay (a two lens system of the same focal distances) is inserted on the beam path to steer the single beam trap across the sample without the beam moving off its position of the back aperture of the microscope objective. A silver mirror is incorporated to redirect the laser beam to the back aperture of the microscope objective. The 100x objective focuses the laser beam to a diffraction limit spot size of 1.08 $\mu$m through the petri dish, about 10 $\mu$m above a glass bottom of the petri dish on which a solution of cells is placed. The petri dish is placed on an automated moving stage.

The laser beam is used for both optical trapping and laser transmission spectroscopy. A long working distance objective (distance focal of 200 mm) is used to collect the transmitted laser light from the sample and couples a Kohler illumination system to the sample plane. The collected transmitted laser beam is focused into a single mode fibre, by a 40 mm distance focal lens, in order to guide the light onto the entrance slit of an Andor (Andor Technology Ltd, 7 Millenium Way, Springvale Business Park, Belfast BT 12 7AL, UK) spectrometer (Shamrock 303i) and the transmittance spectra are detected by an Andor iStar 734 series CCD camera. The iStar camera is air cooled to -20°C in order to reduce the effect of dark current. The images of the trapped cells are viewed through a video camera (Watec, W96N15832 CCD camera) illuminated with the Kohler illumination system.

## Data processing

The transmission spectroscopy data is recorded using Andor's Solis software. The transmission intensity is expressed in terms of photon count which is a measurement of light transmitted through a trapped cell. Prior to the optical trapping transmission spectroscopy experiment, intensity background correction and laser transmitted intensity were recorded in order to subtract the background light from the signal and have a laser intensity reference.

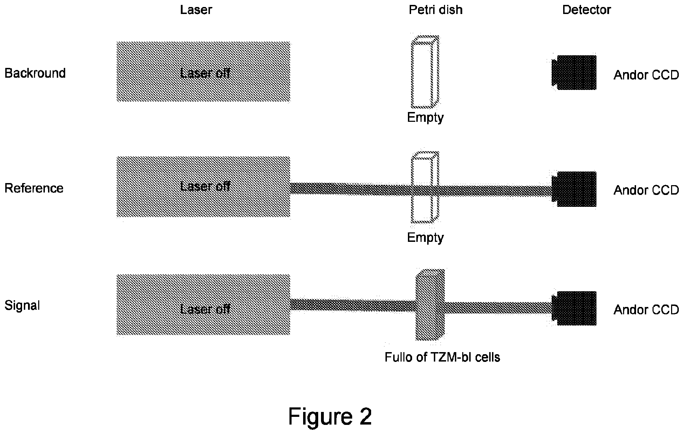

Figure 2 shows a schematic diagram of the procedure to follow in order to record the background, reference and transmitted signal. The intensity background was obtained by recording a dark signal whereby the laser is turned off and no sample is in petri dish. In the same fashion the reference intensity was recorded with laser light turned on and no sample in the petri dish. Similarly the signal was measured with the laser on, and the petri dish full with media and TZM-bl cells whereby one was optically trapped.

In the data processing, the transmission spectra are expressed in transmittance percentage that is given by the equation:

$$I_T\,(\%) = 100 * \left( \frac{I_S - I_B}{I_0} \right)$$

where $I_T$ is transmittance percentage intensity, $I_S$ the signal intensity, $I_B$ the background intensity and $I_0$ the ref-erence intensity.

For each trapped cell, 50 spectra were recorded and an averaged spectrum was calculated and plotted automatically from Andor Solis software. Spectra obtained were exported into OriginPro 8 for data analysis. These procedures were done on ten different trapped cells for the two groups indicated above. The absorption spectra obtained using Ocean Optics software were exported into OriginPro 8 for analysis.

## Results

The present invention is directed at capturing and trapping individual TZM-bl cells (HIV-1 infected and uninfected) within a sample of label-free cells, and performing transmission spectroscopy analysis on a single cell or a plurality of single cells. It is believed that the ability to differentiate between HIV-1 infected and uninfected cells in real-time can be performed by analysing their transmission spectra intensity changes. Firstly, the influence of the cell growth media on the transmittance intensity was studied, where two petri dishes of sample thickness about 1 mm were fitted with 500 $\mu$l of cell growth media with and without the virus respectively.

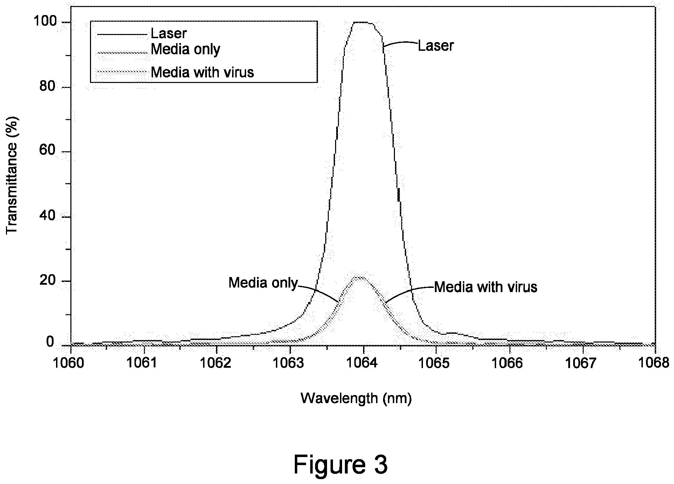

Figure 3 shows transmittance intensity decrease of cell media with and without virus relative to the laser intensity. About an 80% decrease in transmission of the laser intensity was noted. However, transmission spectra observed between cell growth media with and without HIV-1 virus appear relatively similar. Their transmittance percentage remains similar at 20%. This is an indication that the virus in cell growth media does not influence the transmission spectroscopy technique.

To create the optical trap, 60 mW of 1064 nm laser light delivered through a 100x objective was used to trap TZM-bl cells. The Gaussian beam has $M^2 < 1.2$ and a good power stability of 0.108%. The optical trapping system was characterised by using the well-established protocol for trapping spherical silica beads (Lee WM, Reece PJ, Marchington RF, Metzger NK and Dholakia K (2007) Construction and calibration of an optical trap on a fluorescence optical microscope. Nature Protocols 2(12): 3226-3238) and the quality of the trap, q-value, was determined by evaluating the trap efficiency as indicated in literature.

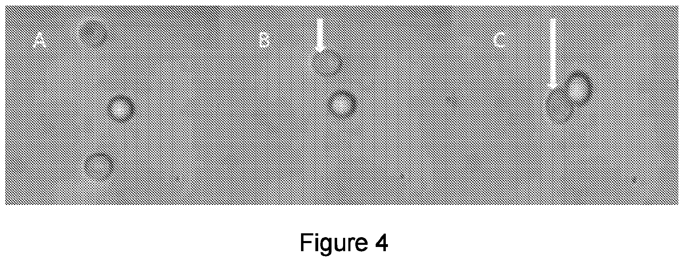

Figure 4 shows a sequence of images of silica beads (from Figure 4a to 4c) as they were being collected. These images exhibit a trapped bead in the centre (Figure 3a) with surrounding untrapped beads. After stage movement (Figure 3b and 3c), the centre bead remains trapped whereas surrounding beads moved as indicated by the arrows. These images were recorded with a stage speed of 60 μm/s. Results obtained here are an indication of a successful trap. This method was repeated for different stage speeds and laser powers in order to determine the q-value. The q-value measured was about 0.045. It is in good agreement with values found in literature that range from 0.03 to 0.1 (Lee *et al.* 2007).

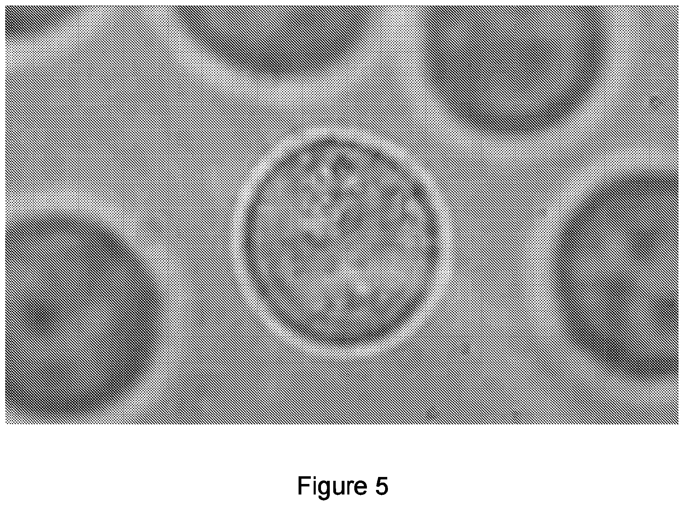

Figure 5 shows a typical single cell trapped in the focus plane whilst the untrapped cells are on the second plane of the image and out of focus.

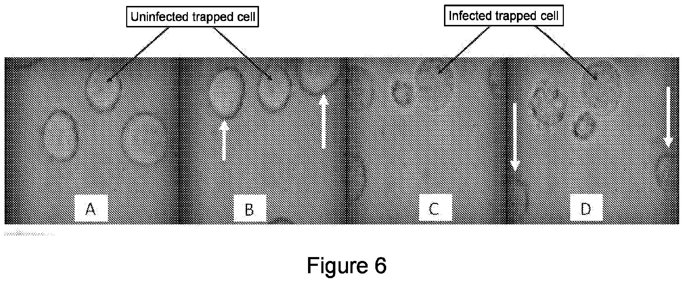

Figure 6 depicts the capability of the optical trapping system to immobilise uninfected and HIV-1 infected TZM-bl single cells. Arrows indicate the movement of untrapped cells. For the TZM-bl HIV-1 infected cells, the morphology and biomolecular composition are different to the uninfected cells. As observed in Figure 6c and 6d, the infected cells presented with granules compared to the uninfected cells in Figure 6a and 6b. It is hypothesised that this difference leads to uninfected and HIV-1 infected TZM-bl cells generating different transmittance spectra.

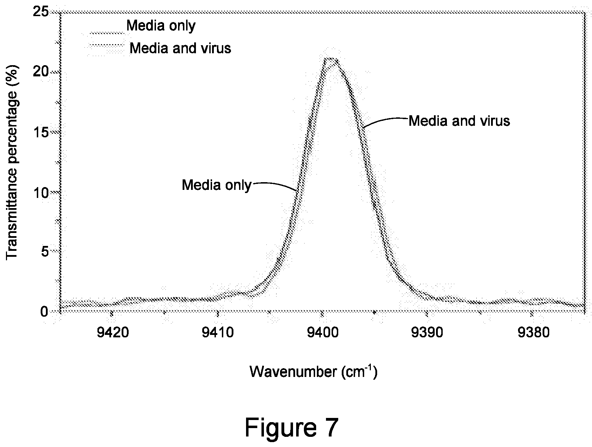

Figure 7 shows transmittance intensity of cell growth media with and without virus. About 20% transmission of the laser intensity was noted for both samples. Transmission spectra observed between cell growth media with and without HIV-1 virus appear relatively similar. Their transmittance percentage remains similar at 20%. Generally, transmission spectra depend strongly on the biochemical composition of the sample, the homogeneity and sample thickness (Mourant JR, Gibson RR, Johnson TM, Carpenter S, Short KW, Yamada YR and Freyer JP (2003) Methods for measuring the infrared spectra of biological cells. Phys Med Biol 48: 243-257). An amount of 25μl of virus was added to cell growth media and showed no difference in spectra. This indicates that the virus in cell growth media does not influence the transmission spectroscopy technique.

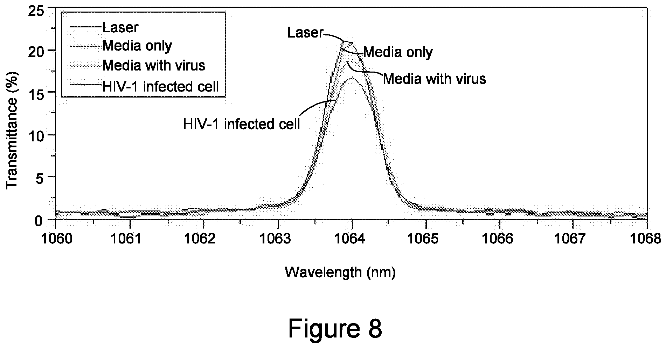

Transmission spectra for separate infected and uninfected single TZM-bl cells are shown in Figure 8. Each spectrum was obtained by taking an average of ten trapped cells. All transmission spectra exhibit similar features and their peaks are at 1064 nm. However, the different TZM-bl cell's spectra reveal variation in the peak amplitude. These results correlate with findings by Ma *et al.* (Ma H, Zhang Y and Ye A (2013) Single-cell discrimination based on optical tweezers Raman spectroscopy. Chinese Science Bulletin, Nano-Biomedical Optoelectronics Materials and Devices 58(21): 2594-2600). Infected and uninfected TZM-bl cells presented a transmittance percentage around 16.77% and 18.80% respectively. Generally, transmittance spectrum depends on parameters such as sample thickness, angle and polarisation of the incident beam, non-uniformity of the cell geometry (Deng JL, Wei Q, Zhang MH, Wang YZ and Li YQ (2005) Study of the effect of alcohol on single human red blood cells using near-infrared laser tweezers Raman spectroscopy. J Raman Spectrosc 36: 257-261) and the absorption coefficient of the sample under investigation.

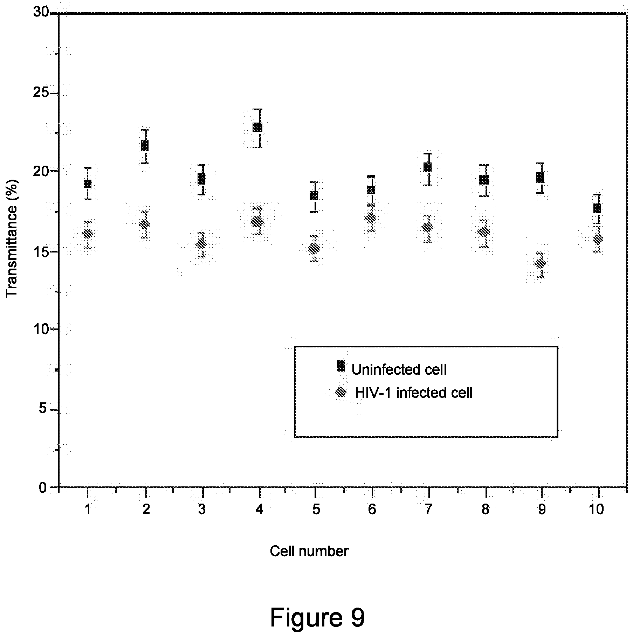

All cells were cultured in the same fashion and uninfected and infected TZM-bl cells samples were prepared at the same stage of the cell cycle. It is assumed that cells were homogenous. In addition, sample thickness (path length) was kept constant. This transmittance percentage difference could be hypothetically attributed to the difference in index of refraction of the two cells (infected and uninfected), hence in their scattering coefficient, as other parameters that play a major role in transmission spectroscopy are constants. Several lines of evidence support that HIV-1 infection can trigger a wide range of reactions in the host cell, including TZM-bl cells. These reactions involve the processing of viral protein into peptide molecules that change the biochemical composition of the TZM-bl cells. As a result, the scattering coefficient will be modified due to a change of index of refraction. Furthermore, Figure 9 demonstrates that transmittance percentage of uninfected TZM-bl trapped cells remains greater than the one for HIV-1 infected trapped cells.

A novel optical trapping transmission spectroscopy technique using near infrared laser has been developed for the real-time spectral analysis and identification of virus infected (i.e. HIV-infected) and uninfected cells in a sample. The combination of the optical trapping system with infrared transmittance spectroscopy is able to provide label-free trapping and transmittance spectra of infected and uninfected TZM-bl cells without any phototoxic and/or thermal damage. The results indicate that there are surprising and significant differences between the transmittance spectra of the HIV-1 infected and uninfected cells. Consequently, the transmission data unexpectedly shows that it is possible to optically differentiate between infected and uninfected cells in real-time and without the requirement of a label or marker. The optical trapping component of the system was tested and calibrated using silica beads and the trap quality factor corresponded well with the range reported in literature.

## Claims

1. An *ex vivo* method of analysing a biological sample comprising a plurality of label-free cells to determine whether or not the sample includes cells that are infected by an HIV virus, the method including:

   optically trapping a label-free cell in the biological sample with an optical beam; and
   spectroscopically investigating the trapped label-free cell to determine in real-time if the trapped label-free cell is infected by an HIV virus, wherein spectroscopically investigating the trapped label-free cell includes determining a transmission value or an absorption value of the label-free cell, and wherein the transmission value or absorption value is indicative of whether or not the label-free cell is infected by the HIV virus.

2. The method of claim 1, wherein spectroscopically investigating the trapped label-free cell is by means of a laser beam.

3. The method of claim 2, wherein optically trapping the label-free cell and spectroscopically investigating the trapped label-free cell are by means of the same laser beam.

4. The method of any one of the preceding claims, wherein near infrared transmission spectroscopy is used to spectroscopically investigate the trapped label-free cells.

5. The method of any one of the preceding claims, wherein optically trapping the label-free cell and spectroscopically investigating the trapped label-free cell are done simultaneously.

6. The method of any one of the preceding claims, wherein the method is implemented iteratively or repetitively in order to investigate a plurality of trapped label-free cells.

7. The method of claim 6, wherein the spectroscopic investigation of the trapped label-free cells indicates a degree of infection of the biological sample.

## Patentansprüche

1. *Ex-vivo*-Verfahren zum Analysieren einer biologischen Probe, die eine Vielzahl von markierungsfreien Zellen enthält, um zu bestimmen, ob die Probe Zellen enthält, die mit einem HIV-Virus infiziert sind, wobei das Verfahren umfasst:

   optisches Einfangen einer markierungsfreien Zelle in der biologischen Probe mit einem optischen Strahl; und
   spektroskopisches Untersuchen der eingefangenen, markierungsfreien Zelle, um in Echtzeit zu bestimmen, ob die eingefangene, markierungsfreie Zelle mit einem HIV-Virus infiziert ist, wobei das spektroskopische Untersuchen der eingefangenen, markierungsfreien Zelle das Bestimmen eines Transmissionswerts oder eines Absorptionswerts der markierungsfreien Zelle umfasst, und wobei der Transmissionswert oder Absorptionswert angibt, ob die markierungsfreie Zelle mit dem HIV-Virus infiziert ist.

2. Verfahren nach Anspruch 1, wobei das spektroskopische Untersuchen der eingefangenen, markierungsfreien Zelle mit einem Laserstrahl erfolgt.

3. Verfahren nach Anspruch 2, wobei das optische Einfangen der markierungsfreien Zelle und das spektroskopische Untersuchen der eingefangenen, markierungsfreien Zelle mit demselben Laserstrahl erfolgt.

4. Verfahren nach einem der vorhergehenden Ansprüche, wobei Nah-Infrarot-Transmissionsspektroskopie verwendet wird, um die eingefangenen, markierungsfreien Zellen spektroskopisch zu untersuchen.

5. Verfahren nach einem der vorhergehenden Ansprüche, wobei das optische Einfangen der markierungsfreien Zelle und das spektroskopische Untersuchen der eingefangenen, markierungsfreien Zelle gleichzeitig erfolgen.

6. Verfahren nach einem der vorhergehenden Ansprüche, wobei das Verfahren iterativ oder repetitiv durchgeführt wird, um eine Vielzahl von eingefangenen, markierungsfreien Zellen zu untersuchen.

7. Verfahren nach Anspruch 6, wobei das spektroskopische Untersuchen der eingefangenen, markierungsfreien Zellen einen Infektionsgrad der biologischen Probe angibt.

## Revendications

1. Procédé *ex vivo* d'analyse d'un échantillon biologique comprenant une pluralité de cellules sans marqueur pour déterminer si l'échantillon comprend ou non des cellules qui sont infectées par un virus VIH, le procédé comprenant :

le piégeage optique d'une cellule sans marqueur dans l'échantillon biologique avec un faisceau optique ; et

l'examen spectroscopique de la cellule sans marqueur piégée pour déterminer en temps réel si la cellule sans marqueur piégée est infectée par un virus VIH, dans lequel l'examen spectroscopique de la cellule sans marqueur piégée comprend la détermination d'une valeur de transmission ou d'une valeur d'absorption de la cellule sans marqueur, et dans lequel la valeur de transmission ou la valeur d'absorption indique si la cellule sans marqueur est infectée ou non par le virus VIH.

2. Procédé selon la revendication 1, dans lequel l'examen spectroscopique de la cellule sans marqueur piégée se fait au moyen d'un faisceau laser.

3. Procédé selon la revendication 2, dans lequel le piégeage optique de la cellule sans marqueur et l'examen spectroscopique de la cellule sans marqueur piégée se font au moyen du même faisceau laser.

4. Procédé selon l'une quelconque des revendications précédentes, dans lequel une spectroscopie de transmission dans le proche infrarouge est utilisée pour l'examen spectroscopique des cellules sans marqueur piégées.

5. Procédé selon l'une quelconque des revendications précédentes, dans lequel le piégeage optique de la cellule sans marqueur et l'examen spectroscopique de la cellule sans marqueur piégée sont effectués simultanément.

6. Procédé selon l'une quelconque des revendications précédentes, dans lequel le procédé est mis en œuvre de manière itérative ou répétitive afin d'examiner une pluralité de cellules sans marqueur piégées.

7. Procédé selon la revendication 6, dans lequel l'examen spectroscopique des cellules sans marqueur piégées indique un degré d'infection de l'échantillon biologique.

Köhler illumination

DM 2

1.5

ANDOR
Spectrometer

0.56 NA

Mitutoyo
LWD objective

PC 1

CCD 1

Beam expander

M1

1.25 NA

100x

Diode Laser

1.1    1.2

M2

1.3    1.4

DM1

Beam steering

BS 50/50    L6    CCD2

M3

L7

PC2

Ocean optics
Spectrometer

Figure 1

| | Laser | Petri dish | Detector |
|---|---|---|---|
| Backround | Laser off | Empty | Andor CCD |
| Reference | Laser off | Empty | Andor CCD |
| Signal | Laser off | Fullo of TZM-bl cells | Andor CCD |

Figure 2

Figure 3

Figure 4

Figure 5

Figure 6

Figure 7

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102014003386 A1

- WO 03093496 A1

### Non-patent literature cited in the description

- Spectroscopic analysis of Kaposi's sarcoma-associated herpesvirus infected cells by Raman tweezers. **HAMDEN et al.** JOUIRNAL OF VIROLOGICAL MET. ELSEVIER BV, 29 June 2005, vol. 129, 145-151
- Raman spectroscopy: the gateway into tomorrow's virology. **LAMBERT PHELPS J et al.** VIROLOGY JOURNAL. BIOMED CENTRAL, 28 June 2006, vol. 3, 51
- **LEE WM ; REECE PJ ; MARCHINGTON RF ; METZGER NK ; DHOLAKIA K.** Construction and calibration of an optical trap on a fluorescence optical microscope. *Nature Protocols,* 2007, vol. 2 (12), 3226-3238

- **MOURANT JR ; GIBSON RR ; JOHNSON TM ; CARPENTER S ; SHORT KW ; YAMADA YR ; FREYER JP.** Methods for measuring the infrared spectra of biological cells. *Phys Med Biol,* 2003, vol. 48, 243-257
- **MA H ; ZHANG Y ; YE A.** Single-cell discrimination based on optical tweezers Raman spectroscopy. *Chinese Science Bulletin, Nano-Biomedical Optoelectronics Materials and Devices,* 2013, vol. 58 (21), 2594-2600
- **DENG JL ; WEI Q ; ZHANG MH ; WANG YZ ; LI YQ.** Study of the effect of alcohol on single human red blood cells using near-infrared laser tweezers Raman spectroscopy. *J Raman Spectrosc,* 2005, vol. 36, 257-261